# EUROPEAN PATENT SPECIFICATION

(11) **EP 2 956 113 B1**
(45) Date of publication and mention of the grant of the patent: **03.04.2019**
(21) Application number: 14703401.1
(22) Date of filing: 10.02.2014
(51) Int. Cl.: A61K 8/25, A61K 8/34, A61K 8/46, A61Q 11/00, A61K 8/73, A61K 8/24

(54) **NON-AQUEOUS ORAL CARE COMPOSITIONS**
NICHTWÄSSRIGE MUNDPFLEGEMITTEL
COMPOSITIONS DE SOINS ORAUX NON AQUEUSES

(30) Priority: 14.02.2013 EP 13155197
(43) Date of publication of application: 23.12.2015
(73) Proprietor: Unilever PLC, London, Greater London EC4Y 0DY (GB); Unilever N.V., 3013 AL Rotterdam (NL)
(72) Inventor: FERRY, Anne-Laure, Sophie, Bebington Wirral Merseyside CH63 3JW (GB); GROVES, Brian, Joseph, Bebington Wirral Merseyside CH63 3JW (GB)
(74) Representative: Tansley, Sally Elizabeth
(86) International application number: PCT/EP2014/052562
(87) International publication number: WO 2014/124904

(56) References cited:
- EP-A1- 0 691 124
- EP-A1- 1 952 801
- WO-A1-01/54657
- WO-A1-2004/032674
- WO-A1-2005/067876
- WO-A1-2012/087326
- WO-A2-95/22958
- US-A1- 2009 087 390

## Description

### Field of the Invention

The present invention is concerned with non-aqueous oral care compositions.

### Background of the Invention

Oral care compositions such as dentifrices typically contain dentally acceptable abrasive, humectant, water, and water-soluble polymer which serves as a thickener and binder for the ingredients. A variety of other ingredients such as flavours, sweeteners, preservatives and fluoride are also utilized at low levels.

However there are many materials which are physically or chemically incompatible with the aqueous environments found in typical dentifrice formulations.

Non-aqueous formulations have been suggested as a way of improving the stability of these materials. For example, US 4,071,615 describes anhydrous dentifrice compositions based on glycerin, thickeners such as hydroxyethylcellulose and carboxymethylcellulose, and silica.

Viscosity profile and flow characteristics are key factors governing ease of processing, product performance and consumer perception of a dentifrice. A problem with non-aqueous formulations such as those disclosed in US 4,071,615 is that they do not behave rheologically like a typical aqueous dentifrice. In particular they may exhibit insufficient consistency with a "runny" character, leading to manufacturing difficulties and reduced acceptance amongst consumers. One way of improving the rheological behaviour of anhydrous dentifrice formulations is described in WO2011/160996. According to this publication, improved microstructure, ease of processing and sensory properties are obtained by structuring the liquid continuous phase with a crystalline structuring agent, such as one or more solid polyethylene glycols having a melting point of 25°C or above.

EP 1 952 801 and WO95l22958 describe oral care compositions comprising calcium glycerophosphate.

WO2005/067876 describes oral care compositions comprising an abrasive system comprising a combination of amorphous silica and crystalline aluminosilicate. However, the preparation process described in WO2011/160996 requires carefully controlled heating and cooling stages in order to ensure the formation of a crystalline network with the requisite structuring properties.

The present inventors have found that the above problems may be solved by the incorporation of xanthan gum as a structurant.

WO97/28783 describes xanthan gum as an ingredient in an oral formulation based on 87 to 99% by weight anhydrous glycerin. The formulation according to WO97/28783 is a stable stannous fluoride gel which can be extruded onto the bristles of a toothbrush. However, the formulation is thickened with a polyethylene glycol 1000 thickener in an amount of at least 3% by weight. It is also not suitable for cleaning the surfaces of the oral cavity.

### Summary of the Invention

The present invention provides a non-aqueous oral care composition suitable for cleaning the surfaces of the oral cavity, the composition comprising:
(a) from 20 to 90wt% (by weight by based on the total weight of the composition) of one or more organic polyols having 3 or more hydroxyl groups in the molecule;
(b) from 3 to 75wt% (by weight based on the total weight of the oral care composition) of one or more particulate abrasive cleaning agents;
(c) from 0.2 to 5wt% (by weight based on the total weight of the oral care composition) of one or more surfactants;
(d) from 0.01 to 5wt% (by weight based on the total weight of the oral care composition) of xanthan gum, and
(e) comprises a mixture of a calcium source and a phosphate source in which the calcium source is calcium silicate in which the phosphate source is a mixture of trisodium phosphate and monosodium dihydrogen phosphate;
   In which one or more particulate abrasive cleaning agents are selected from abrasive silicas and mixtures thereof;
   Surprisingly, the oral care composition of the invention is easy to manufacture without the need for careful temperature control. It is also unexpectedly stable and cost-effective, with suitable viscosity characteristics despite its non-aqueous nature.

### Detailed Description of the Invention

The composition of the invention is non-aqueous. The term "non-aqueous" in the context of the present invention generally means that water is not deliberately added to the composition in any significant quantity. However, the term "non-aqueous" does not mean that small amounts of water cannot be present, for example as a consequence of its association with hygroscopic raw materials. Accordingly, for the purposes of this invention, the term "non-aqueous" generally means that water is present in an amount no greater than about 5wt%, more preferably no greater than about 3wt% (by weight based on the total weight of the composition).

The composition of the invention comprises from 20 to 90wt% (by weight by based on the total weight of the composition) of one or more organic polyols having 3 or more hydroxyl groups in the molecule.

Preferred organic polyols having 3 or more hydroxyl groups in the molecule (hereinafter termed "organic polyols"). for use in the invention include glycerol, sorbitol, xylitol, mannitol, lactitol, maltitol, erythritol, and hydrogenated partially hydrolyzed polysaccharides. The most preferred organic polyol is glycerol. Mixtures of any of the above described materials may also be used.

The composition of the invention is most preferably organic polyol-based. In the context of the present invention, the term "organic polyol-based" generally means that the composition is not oil-based or water-based, but instead organic polyol (as defined above) forms a liquid continuous phase in which the particulate ingredients of the composition (such as the one or more particulate abrasive cleaning agents) are dispersed.

Ideally the composition of the invention is glycerol-based.

The amount of organic polyol in the composition of the invention suitably ranges from 35 to 90wt%, preferably from 45 to 80wt% (by total weight organic polyol based on the total weight of the composition). More preferably the composition contains from 60 to 75wt% glycerol based on the total weight of the composition.

The composition of the invention comprises a particulate abrasive cleaning agent in an amount of from 3 to 75wt% (by weight based on the total weight of the oral care composition). Particulate abrasive cleaning agents for use in the invention are selected from abrasive silicas such as silica xerogels, hydrogels and aerogels and precipitated particulate silicas and mixtures thereof.

Mixtures of any of the above described materials may also be used.

The composition of the invention comprises one or more surfactants in an amount of from 0.2 to 5wt% (by weight based on the total weight of the oral care composition) Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Preferred surfactants for use in the invention are selected from sodium lauryl sulfate, sodium lauryl sulfoacetate, cocamidopropyl betaine, sodium alpha olefin sulfonate, dioctyl sodium sulfosuccinate, sodium dodecyl benzene sulfonate and mixtures thereof.

Mixtures of any of the above described materials may also be used.

The composition of the invention comprises xanthan gum in an amount of from 0.01 to 5wt% (by weight based on the total weight of the oral care composition).

Xanthan gum is a fermentation product prepared by action of the bacteria of the genus *Xanthomonas* upon carbohydrates. Four species of *Xanthomonas,* namely *X.campestris, X. phaseoli, X.malvocearum* and *X.carotae* are reported in the literature to be the most efficient gum producers.

Xanthan gum may be generally characterised as an anionic heteropolysaccharide, with a primary structure consisting of repeating pentasaccharide units formed by two glucose units, two mannose units, and a glucuronic acid unit. These repeating pentasaccharide units give xanthan gum its characteristic backbone, which consists of (1→4) β-D-glucopyranosyl units substituted at C-3 on every other glucose residue with a charged trisaccharide sidechain. The trisaccharide sidechain consists of a D-glucuronic acid unit between 2 D-mannose units. Slightly less than half (about 40%) of the terminal D-mannose residues contain a pyruvic acid residue linked via keto groups to the four and six positions, and the D-mannose linked to the main chain mostly contains an acetyl group at position O-6. Some side chains may be missing. The acetate and pyruvate contents are variable on the side chain, and depend on the bacterial strains and on the fermentation conditions used to produce the gum.

Xanthan gum generally has a molecular weight of from 1 million to 50 million. Its viscosity generally ranges from 850 to 1,700 mPa.s (when measured at 25°C using a 1% solution of the gum in 1% KCI, on a viscometer of the Brookfield LV type, at 60 rpm using Spindle No. 3).

Xanthan gum is available from several commercial suppliers such a RT Vanderbilt Company and CP Kelco. Examples of suitable xanthan gums are Keltrol®, Keltrol® F, Keitrol® T, Keltrol® TF, Xantural® 180 and Vanzan® NF.

The amount of xanthan gum in the composition of the invention preferably ranges from 0.05 to 1.5wt%, more preferably from 0.1 to 0.9wt% (by weight based on the total weight of the oral care composition).

In addition to the xanthan gum as described above, other polymeric thickening agents may be present in the composition of the invention. Such additional polymeric thickening agents may be of natural or synthetic origin, with a molecular weight typically ranging from 1,000 up to about 5,000,000. Examples of such materials include carboxyvinyl polymers (such as polyacrylic acids cross-linked with polyallyl sucrose or polyallyl pentaerythritol), hydroxyalkyl cellulose derivatives (such as hydroxyethyl cellulose, hydroxypropyl cellulose, hydroxyethylpropyl cellulose, hydroxybutylmethyl cellulose and hydroxypropylmethyl cellulose), water soluble salts of cellulose ethers (such as sodium carboxymethyl cellulose and sodium carboxymethyl hydroxyethyl cellulose, polyvinylpyrrolidone, polyethylene glycols, and mixtures thereof.

However, we have found that the xanthan gum as described above is a surprisingly effective structurant for the composition of the invention. Accordingly it is not generally necessary to include significant quantities of additional polymeric thickening agents. Preferably the level of such materials is no more than 0.5wt%, more preferably no more than 0.1wt%, most preferably no more than 0.01wt%, ideally 0wt% (by total weight additional polymeric thickening agents based on the total weight of the composition).

### Product Form and Optional Ingredients

The oral care composition of the invention is used to clean the surfaces of the oral cavity.

Accordingly, preferred product forms for compositions of the invention are those which are suitable for brushing and/or rinsing the surfaces of the oral cavity.

The composition of the invention is most preferably in the form of a dentifrice. The term "dentifrice" denotes an oral composition which is used to clean the surfaces of the oral cavity. Such a composition is not intentionally swallowed for purposes of systemic administration of therapeutic agents, but is applied to the oral cavity, used to treat the oral cavity and then expectorated. Typically such a composition is used in conjunction with a cleaning implement such as a toothbrush, usually by applying it to the bristles of the toothbrush and then brushing the accessible surfaces of the oral cavity.

Preferably the dentifrice is in the form of an extrudable semi-solid such as a cream, paste or gel (or mixture thereof).

A composition according to the invention (such as a dentifrice) will generally contain further ingredients to enhance performance and/or consumer acceptability, in addition to the ingredients specified above.

For example, being non-aqueous, the composition of the invention is particularly suitable as a vehicle for oral care actives which may be physically or chemically incompatible with water, or which may function less efficiently in an aqueous environment.

Specific examples of oral care actives which may be particularly suitable for inclusion in the compositions of the invention are:
water-soluble or sparingly water-soluble sources of zinc ions such as zinc chloride, zinc acetate, zinc gluconate, zinc sulphate, zinc fluoride, zinc citrate, zinc lactate, zinc oxide, zinc monoglycerolate, zinc tartrate, zinc pyrophosphate and zinc maleate;
oral care enzyme systems such as hydrogen peroxide producing enzyme systems (e.g. the oxidoreductase enzyme glucose oxidase), amyloglucosidase, dextranase and/or mutanase, (optionally in the presence of zinc ion providing compounds and/or 8- hydroxyquinoline derivatives), lactoperoxidase, lactoferrin, lysozyme and mixtures thereof;
fluoride sources such as sodium fluoride, stannous fluoride, sodium monofluorophosphate, zinc ammonium fluoride, tin ammonium fluoride, calcium fluoride, cobalt ammonium fluoride and mixtures thereof;
plant-derivable antioxidants such as flavonoid, catechin, polyphenol, and tannin compounds and mixtures thereof;
antioxidant vitamins such as tocopherols and/or derivatives thereof, ascorbic acid and/or derivatives thereof and mixtures thereof.

Compositions of the invention comprise a class of oral care actives for the remineralisation of teeth. The term "remineralisation" in the context of the present invention means the *in situ* generation of hydroxyapatite on teeth.

The remineralisation of teeth actives comprise a mixture of a calcium source and a phosphate source which, when delivered to the teeth results in the *in situ* generation of hydroxyapatite on teeth.

The alcium source that may be used in this context (hereinafter termed "remineralising calcium sources") is calcium silicate.

The amount of remineralising calcium source(s) (e.g. calcium silicate) in the composition of the invention typically ranges from 1 to 30%, preferably from 5 to 20% by total weight remineralising calcium source based on the total weight of the oral care composition.

The remineralising phosphate source is a mixture of trisodium phosphate and sodium dihydrogen phosphate.

The amount of remineralising phosphate source(s) (e.g. trisodium phosphate and sodium dihydrogen phosphate) in the composition of this invention typically ranges from 2 to 15%, preferably from 4 to 10% by total weight remineralising phosphate source based on the total weight of the oral care composition.

Mixtures of any of the above described materials may also be used.

Compositions of the present invention may also contain further optional ingredients customary in the art such as anticalculus agents, buffers, flavouring agents, sweetening agents, colouring agents, opacifying agents, preservatives, antisensitivity agents, antimicrobial agents and the like.

### Process

The invention also provides a process of preparing a non-aqueous oral care composition suitable for cleaning the surfaces of the oral cavity, the process comprising the steps of forming a mixture comprising ingredients (a) to (d) (as defined above).

A preferred process according of the invention comprises the following steps:
(i) forming a mixture of the organic polyol (a) and the xanthan gum (d) and heating the mixture (preferably to a temperature above 50°C) to disperse the xanthan gum;
(ii) adding powdered ingredients (preferably the abrasive cleaning agent (b) and/or the surfactant (c)) to the mixture obtained in (i), and
(iii) cooling the mixture obtained in (ii).

Preferably, other optional ingredients selected from fluoride sources and/or agents for the remineralisation of teeth (such as calcium silicate, as further described above) are added between steps (i) and (ii).

Other optional ingredients such as flavourings may suitably be added after step (iii) to form the final non-aqueous oral care composition.

The invention is further illustrated with reference to the following, non-limiting Example.

### EXAMPLE

### Example 1

The following formulation illustrates a dentifrice composition according to the invention:

| **Ingredient** | **(wt%)** |
|---|---|
| Glycerol (99.5% a.i.) | 67.4 |
| Xanthan gum | 0.5 |
| Trisodium phosphate anhydrous | 3.5 |
| Monosodium dihydrogen phosphate | 3.2 |
| Sodium monofluorophosphate | 1.1 |
| Sodium saccharin | 0.2 |
| Flavour | 1.1 |
| Calcium silicate | 15 |
| Sorbosil®AC77 (ex PQ Corp.) | 6.0 |
| Sodium lauryl sulphate | 2.000 |

Viscosity measurements were performed on the above formulation using a rheometer, and a rheological profile plotted in the form of viscosity versus shear stress. The rheological profile obtained showed a relatively high viscosity plateau at low shear stress (i.e. between 10,000 and 100,000 Pa.s at shear stresses of 10 Pa or less), and a shear-thinning behaviour with a relatively low viscosity at high shear stress (i.e between 10 and 100 Pa.s at shear stresses between 1000 and 10000 Pa). These rheological characteristics are highly desirable for dentifrice compositions and compare well with conventional aqueous dentifrices.

## Claims

1. A non-aqueous oral care composition suitable for cleaning the surfaces of the oral cavity, the composition comprising:
(a) from 20 to 90wt% (by weight by based on the total weight of the composition) of one or more organic polyols having 3 or more hydroxyl groups in the molecule;
(b) from 3 to 75wt% (by weight based on the total weight of the oral care composition) of one or more particulate abrasive cleaning agents;
(c) from 0.2 to 5wt% (by weight based on the total weight of the oral care composition) of one or more surfactants;
(d) from 0.01 to 5wt% (by weight based on the total weight of the oral care composition) of xanthan gum;
(e) comprises a mixture of a calcium source and a phosphate source in which the calcium source is calcium silicate in which the phosphate source is a mixture of trisodium phosphate and monosodium dihydrogen phosphate; and
in which one or more particulate abrasive cleaning agents are selected from abrasive silicas and mixtures thereof.

2. A composition according to claim 1, in which the one or more organic polyols having 3 or more hydroxyl groups in the molecule is glycerol and the level of glycerol is from 60 to 75wt% based on the total weight of the composition.

3. A composition according to any one of claims 1 to 2, in which the amount of xanthan gum ranges from 0.1 to 0.9wt% (by weight based on the total weight of the oral care composition).

4. A composition according to any one of claims 1 to 3, in which the amount of additional polymeric thickening agents (with molecular weight ranging from 1,000 to 5,000,000, and which are selected from carboxyvinyl polymers, hydroxyalkyl cellulose derivatives, water soluble salts of cellulose ethers, polyvinylpyrrolidone, polyethylene glycols and mixtures thereof) is no more than 0.01wt% (by total weight additional polymeric thickening agents based on the total weight of the composition).

5. A composition according to any one of claims 1 to 4, which is in the form of a dentifrice.

6. A process of preparing a non-aqueous oral care composition suitable for cleaning the surfaces of the oral cavity according to any preceding claim, the process comprising the steps of forming a mixture comprising:
(a) from 20 to 90wt% (by weight by based on the total weight of the composition) of one or more organic polyols having 3 or more hydroxyl groups in the molecule;
(b) from 3 to 75wt% (by weight based on the total weight of the oral care composition) of one or more particulate abrasive cleaning agents in which one or more of the particulate abrasive agents are selected from abrasive silicas and mixtures thereof;
(c) from 0.2 to 5wt% (by weight based on the total weight of the oral care composition) of one or more surfactants.
(d) from 0.01 to 5wt% (by weight based on the total weight of the oral care composition) of xanthan gum and
(e) a mixture of a calcium source and a phosphate source in which the calcium source is calcium silicate in which the phosphate source is a mixture of trisodium phosphate and monosodium dihydrogen phosphate.

## Patentansprüche

1. Nichtwässrige Mundpflegezusammensetzung, die zum Reinigen der Oberflächen der Mundhöhle geeignet ist, wobei die Zusammensetzung umfasst:
(a) von 20 bis 90 Gew.-% (gewichtsmäßig, bezogen auf das Gesamtgewicht der Zusammensetzung) an einem oder mehreren organischen Polyolen, die 3 oder mehr Hydroxygruppen im Molekül aufweisen;
(b) von 3 bis 75 Gew.-% (gewichtsmäßig, bezogen auf das Gesamtgewicht der Mundpflegezusammensetzung) an einem oder mehreren teilchenförmigen abrasiven Reinigungsmitteln;
(c) von 0,2 bis 5 Gew.-% (gewichtsmäßig, bezogen auf das Gesamtgewicht der Mundpflegezusammensetzung) an einem oder mehreren Tensiden;
(d) von 0,01 bis 5 Gew.-% (gewichtsmäßig, bezogen auf das Gesamtgewicht der Mundpflegezusammensetzung) Xanthangummi;
(e) umfasst eine Mischung einer Calciumquelle und einer Phosphatquelle, in welcher die Calciumquelle Calciumsilikat ist, in welcher die Phosphatquelle eine Mischung von Trinatriumphosphat und Mononatriumdihydrogenphosphat ist; und
in welcher ein oder mehrere teilchenförmige abrasive Reinigungsmittel aus abrasiven Kieselerden und Mischungen davon ausgewählt sind.

2. Zusammensetzung nach Anspruch 1, in welcher die ein oder mehreren organischen Polyole, die 3 oder mehr Hydroxygruppen im Molekül aufweisen, Glycerin darstellen und der Anteil des Glycerins von 60 bis 75 Gew.-%, bezogen auf das Gesamtgewicht der Zusammensetzung, beträgt.

3. Zusammensetzung nach irgendeinem der Ansprüche 1 bis 2, in welcher sich die Menge des Xanthangummis von 0,1 bis 0,9 Gew.-% (gewichtsmäßig, bezogen auf das Gesamtgewicht der Mundpflegezusammensetzung) erstreckt.

4. Zusammensetzung nach irgendeinem der Ansprüche 1 bis 3, in welcher die Menge an zusätzlichen polymeren Verdickungsmitteln (mit einem Molekulargewicht, das sich von 1.000 bis 5.000.000 erstreckt, und welche aus Carboxyvinylpolymeren, Hydroxyalkylcellulosederivaten, wasserlöslichen Salzen von Celluloseethern, Polyvinylpyrrolidon, Polyethylenglycolen und Mischungen davon ausgewählt sind) nicht mehr als 0,01 Gew.-% (bezogen auf das Gesamtgewicht der zusätzlichen polymeren Verdickungsmittel, bezogen auf das Gesamtgewicht der Zusammensetzung) beträgt.

5. Zusammensetzung nach irgendeinem der Ansprüche 1 bis 4, welche in Form eines Zahnputzmittels vorliegt.

6. Verfahren zur Herstellung einer nichtwässrigen Mundpflegezusammensetzung, die zum Reinigen der Oberflächen der Mundhöhle geeignet ist, nach irgendeinem vorhergehenden Anspruch, wobei das Verfahren die Schritte des Ausbildens einer Mischung umfasst, umfassend:
(a) von 20 bis 90 Gew.-% (gewichtsmäßig, bezogen auf das Gesamtgewicht der Zusammensetzung) an einem oder mehreren organischen Polyolen, die 3 oder mehr Hydroxygruppen im Molekül aufweisen;
(b) von 3 bis 75 Gew.-% (gewichtsmäßig, bezogen auf das Gesamtgewicht der Mundpflegezusammensetzung) an einem oder mehreren teilchenförmigen abrasiven Reinigungsmitteln, bei denen ein oder mehrere der teilchenförmigen abrasiven Mittel aus abrasiven Kieselerden und Mischungen davon ausgewählt werden;
(c) von 0,2 bis 5 Gew.-% (gewichtsmäßig, bezogen auf das Gesamtgewicht der Mundpflegezusammensetzung) an einem oder mehreren Tensiden;
(d) von 0,01 bis 5 Gew.-% (gewichtsmäßig, bezogen auf das Gesamtgewicht der Mundpflegezusammensetzung) Xanthangummi; und
(e) eine Mischung einer Calciumquelle und einer Phosphatquelle, in welcher die Calciumquelle Calciumsilikat ist, in welcher die Phosphatquelle eine Mischung von Trinatriumphosphat und Mononatriumdihydrogenphosphat ist.

## Revendications

1. Composition non-aqueuse pour le soin oral appropriée pour nettoyer les surfaces de la cavité orale, la composition comprenant :
(a) de 20 à 90 % en masse (en masse sur la base de la masse totale de la composition) d'un ou plusieurs polyols organiques ayant 3 groupes hydroxyle ou plus dans la molécule ;
(b) de 3 à 75 % en masse (en masse sur la base de la masse totale de la composition pour le soin oral) d'un ou plusieurs agents nettoyants abrasifs particulaires ;
(c) de 0,2 à 5 % en masse (en masse sur la base de la masse totale de la composition pour le soin oral) d'un ou plusieurs tensioactifs ;
(d) de 0,01 à 5 % en masse (en masse sur la base de la masse totale de la composition pour le soin oral) de gomme xanthane ;
(e) comprend un mélange d'une source de calcium et d'une source de phosphate dans lequel la source de calcium est le silicate de calcium dans lequel la source de phosphate est un mélange de phosphate de trisodium et de dihydrogénophosphate de monosodium ; et
dans laquelle un ou plusieurs agents nettoyants abrasifs particulaires sont choisis parmi des silices abrasives et des mélanges de celles-ci.

2. Composition selon la revendication 1, dans laquelle le un ou plusieurs polyols organiques présentant 3 groupes hydroxyle ou plus dans la molécule est le glycérol et la teneur en glycérol est de 60 à 75 % en masse sur la base de la masse totale de la composition.

3. Composition selon l'une quelconque des revendications 1 à 2, dans laquelle la quantité de gomme xanthane est de 0,1 à 0,9 % en masse (en masse sur la base de la masse totale de la composition pour le soin oral).

4. Composition selon l'une quelconque des revendications 1 à 3, dans laquelle la quantité d'agents épaississants polymères supplémentaires (avec une masse moléculaire de 1 000 à 5 000 000, et qui sont choisis parmi des polymères de carboxyvinyle, des dérivés d'hydroxyalkylcellulose, des sels solubles dans l'eau d'éthers de cellulose, la polyvinylpyrrolidone, des polyéthylène glycols et des mélanges de ceux-ci) n'est pas supérieure à 0,01 % en masse (en masse totale d'agents épaississants polymères supplémentaires sur la base de la masse totale de la composition).

5. Composition selon l'une quelconque des revendications 1 à 4, laquelle est dans la forme d'un dentifrice.

6. Procédé de préparation d'une composition non-aqueuse pour le soin oral appropriée pour nettoyer les surfaces de la cavité orale selon l'une quelconque des revendications précédentes, le procédé comprenant les étapes de formation d'un mélange comprenant :
(a) de 20 à 90 % en masse (en masse sur la base de la masse totale de la composition) d'un ou plusieurs polyols organiques ayant 3 groupes hydroxyle ou plus dans la molécule ;
(b) de 3 à 75 % en masse (en masse sur la base de la masse totale de la composition pour le soin oral) d'un ou plusieurs agents nettoyants abrasifs particulaires dans lequel un ou plusieurs des agents nettoyants particulaires sont choisis parmi des silices abrasives et des mélanges de celles-ci ;
(c) de 0,2 à 5 % en masse (en masse sur la base de la masse totale de la composition pour le soin oral) d'un ou plusieurs tensioactifs ;
(d) de 0,01 à 5 % en masse (en masse sur la base de la masse totale de la composition pour le soin oral) de gomme xanthane et
(e) un mélange d'une source de calcium et d'une source de phosphate dans lequel la source de calcium est le silicate de calcium dans lequel la source de phosphate est un mélange de phosphate de trisodium et de dihydrogénophosphate de monosodium.
